# EUROPEAN PATENT SPECIFICATION

(11) **EP 3 703 650 B1**
(45) Date of publication and mention of the grant of the patent: **27.04.2022**
(21) Application number: 18797268.2
(22) Date of filing: 10.10.2018
(51) Int. Cl.: A61K 8/39, A61K 8/06, A61K 8/02, A61Q 1/14, A61Q 5/12, A61Q 5/02, A61Q 15/00, A61Q 17/04, A61Q 1/06, A61Q 5/06, A61Q 19/00, C07C 69/28, C07C 43/11

(54) **1,3-PROPYLENE ETHER DERIVED COMPOUNDS FOR PERSONAL CARE**
1,3-PROPYLENE ETHER ABGELEITETE VERBINDUNGEN FÜR DIE KÖRPERPFLEGE
COMPOSÉS DÉRIVÉS DE 1,3-PROPYLÈNE ETHER POUR SOIN CORPOREL

(30) Priority: 01.11.2017 US 201762579991 P
(43) Date of publication of application: 09.09.2020
(73) Proprietor: Croda, Inc., Plainsboro, NJ 08536 (US)
(72) Inventor: GUNDERMAN, Erik, Edison, NJ 08837 (US); SAPORITO, Paul, Joseph, Edison, NJ 08837 (US); MATT, Joseph, Nicholas, Edison, NJ 08837 (US); PEREIRA, Abel, Goncalves, Edison, NJ 08837 (US)
(74) Representative: Craven, Ian
(86) International application number: PCT/US2018/055178
(87) International publication number: WO 2019/089202

(56) References cited:
- WO-A1-2008/080892
- WO-A2-03/013439
- WO-A2-03/032919
- DE-A1- 1 694 630
- DE-A1- 3 122 693
- JP-A- 2005 350 366
- US-A- 3 115 422
- US-A- 3 657 126
- SIRICHAIWAT CHAWANEE ET AL: "Target guided synthesis of 5-benzyl-2,4-diamonopyrimidines: Their antimalarial activities and binding affinities to wild type and mutant dihydrofolate reductases from Plasmodium falciparum", JOURNAL OF MEDICINAL CHEMISTRY, AMERICAN CHEMICAL SOCIETY, vol. 47, no. 2, 15 January 2004 (2004-01-15), pages 345-354, XP002451169, ISSN: 0022-2623, DOI: 10.1021/JM0303352

## Description

This application is related, and claims the benefit of priority of, U.S. Provisional Application No. 62/579,991, titled COMPOUNDS SUITABLE FOR PERSONAL CARE AND COSMETIC USES, filed on 1 November 2017.

### Field of the Invention

The present invention is as defined in the appended claims and relates to novel compounds, ether and ester derivatives of polytrimethylene ether glycol, personal care products containing them, methods of producing them, and their use as ingredients for personal care and/or cosmetic formulations, in particular as agents to provide sensory and pigment wetting properties.

### Background of the Invention

Known polyalkylene ether glycols include polyethylene glycol, poly-1,2-propylene ether glycol, 1,3-propylene ether glycol, polytetramethylene ether glycol, polyhexamethylene ether glycol and copolymers thereof. Examples of commercially important polyether glycols include polyethylene glycol, poly(1,2-propylene glycol), ethylene oxide/propylene oxide copolyols, and polytetramethylene ether glycol.

The most widely used polyether glycol is poly(1,2-propylene glycol) (PPG) because of its low cost. This polymer is non-crystalline, liquid at room temperature and easy to handle. However, PPG is derived from a non-renewable reactive epoxide and has secondary hydroxyl end groups.

Polytrimethylene ether glycol or poly(1,3-propylene glycol) (PPDn) can be produced from non-epoxide 1,3-propanediol, which can be advantageously derived from a renewable source. Polytrimethylene ether glycols have primary hydroxyl groups, have low melting points and are highly flexible.

Polytrimethylene ether glycols have been known for a number of years, as well as fatty acid mono or di-ester derivatives. They are used in different technical fields, especially as functional fluids.

In the personal care and cosmetic fields, polypropanediol-5 stearate is used as a surfactant or emulsifying formulation agent.

DE 31 22 693 A1 discloses the use of polyisocyanate-polyol mixtures to stabilize rock and coal formations. Polyether polyols based on propylene glycol and propylene oxide with OH numbers of 205 and 250 are used.

US 3 657 126 A discloses an oil and water-base lubricant useful in drawing and ironing operations as well as in spinning and surfacing. Polymeric mixed diesters of tripropylene or triethylene glycol having 1 to 10 carbons in the acid radicals are used (generic formula RCOO(CH2)"O(CH2)"OOCR' wherein R and R' are different alkyl groups each having 1 to 9 carbons and n is 2 or 3). Representative mixed diesters of this formula are tripropylene glycol caprylate caprate, triethylene glycol caprylate caprate, triethylene glycol butyrate caprylate and tripropylene glycol propionate caproate.

Sirichaiwat Chawanne et al. in "Target guided synthesis of 5-benzyl-2,4-diamonopyrimidines: Their antimalarial activities and binding affinities to wild type and mutant dihydrofolate reductases from Plasmodium falciparum", Journal of medicinal chemistry, American Chemimal Society, vol. 47, no. 2,15 January 2004 (2004-01-15), pages 345-354, disclose a compound of formula X-(OCH2CH2CH2)2-OR, wherein X and R are cyclic aromatic hydrocarbyls.

US 3 115 422 A discloses a treatment of metals to increase the fatigue life thereof. The dipropyleneglycol dipelargonate is mentioned a list of lubricants which were used to pretreat bearing balls.

DE 16 94 630 A1 discloses compositions of plasticized vinyl resins resistant to staining. Plastifiers are disclosed having the formula R-Ph-O-(CH2-CH2-O)n-OC(O)R3 are disclosed wherein R, R1 and R2 are hydrogen or methyl, R3 is an aliphatic radical, satured or not, branched or not, having 2-5 carbon atoms, and n is 1, 2 or 3.

JP 2005 350366 A discloses a water-in-oil type skin care composition for external use containing a high-molecular copolymer comprising an acrylic acid-based monomer (A), a polyoxyalkylene group-containing monomer (B) and a organopolysiloxane-containing monomer (C) as constituent monomers. The monomer (B) has the general formula CH2=C(R3)-C(O)-O-(R4-O)I-R5, wherein R3 is hydrogen or a hydrocarbon group having 1 to 3 carbon atoms, R4 is a divalent hydrocarbon group having 1 to 4 carbon atoms, R5 is hydrogen or a hydrocarbon group having 1 to 24 carbon atoms, and I represents an integer from 1 to 50.

WO03/013439 discloses anti-irritants, WO2008/080892 discloses UV absorber formulations and WO03/032919 discloses esters of aromatic alkoxylated alcohols and fatty carboxylic acids.

The aim of the present invention is to propose new compound derivatives of polytrimethylene ether glycol, in particular for the field of personal care products.

Traditional cosmetic pigments are made in different ways from thermal decomposition, extraction and other filtration processes from solutions to produce a dry powder. However, these dry pigmentary powders come in multiple forms of particle aggregates/agglomerates that introduce difficulties in color properties when evaluated. Hence, they are oftentimes difficult for cosmetics chemists to formulate/stabilize on various product forms like emulsions, anhydrous sticks or powder formulations. These dry powders need to be fully dispersed and their particle size reduced as much as possible to see the full development of color. Pigment dispersion has been used by cosmetic scientists as a beneficial way to introduce/incorporate dry powders into their respective formulations.

Dispersion is the process of wetting, separating and distributing pigment particles in a medium. It requires intense energy input through high sheering in liquids or pulverization in powders. It can also require specialized equipment like high sheer mixers, 3 roll mills, etc.

Some of the benefits of using dispersions are: the full development of color, enhanced color intensity, the prevention of agglomeration or reagglomeration, the prevention of pigment striations on product formulations, cost efficacy and the ease of incorporation in various product forms.

### Summary of the Invention

The objective of the present invention is to address these and other disadvantages associated with the prior art. A specific objective of the invention is to propose a compound or composition having interesting properties on dispersing inorganic pigments/dyes.

Accordingly, a first aspect of the present invention provides for a compound of formula 1:

X-(OCH₂CH₂CH₂)ₙ-OR (1)

wherein:
n is an integer from 2 to 15,
R is a C₆ to C₃₆ hydrocarbyl group,
X is R or R₁C(O), and
   R₁ is a C₂ to C₃₆ hydrocarbyl group, and
wherein R and R₁ groups are alkyl groups.

According to a second aspect of the present invention, there is provided a composition comprising a mixture of two or more compounds according to the first aspect.

According to a third aspect of the present invention, there is provided a method of producing the compound of the first aspect of the invention or the composition of the second aspect of the invention.

A fourth aspect of the present invention provides for a personal care product comprising the compound of the first aspect of the invention or the composition of the second aspect of the invention.

A fifth aspect of the present invention provides for the use of a compound according to the first aspect of the invention, or a composition according to the second aspect of the invention, in a personal care product.

All of the features described herein may be combined with any of the above aspects, in any combination.

The present invention is based in part on the recognition by the inventors that a compound or composition of the first or second aspect of the invention, respectively, has advantageous properties due to its particular combination of components.

It has surprisingly been discovered that capping polytrimethylene ether glycol with a fatty alcohol group at least on one end side, the other end side being capped with an ester group, provides new ether derivative compounds, which can be named "ester-PPDn-ether" or "ether-PPDn-ether", possessing desirable rheological properties for formulation purposes, in particular pigment wetting properties, and/or sensory properties. Such properties are presented through the examples given below.

### Detailed description of the invention

It will be understood that any upper or lower quantity or range limit used herein may be independently combined.

It will be understood that, when describing the number of carbon atoms in a substituent group (e.g. 'C₁ to C₆'). the number refers to the total number of carbon atoms present in the substituent group, including any present in any branched groups. Additionally, when describing the number of carbon atoms in, for example fatty acids, this refers to the total number of carbon atoms including the one at the carboxylic acid, and any present in any branch groups.

Many of the chemicals which may be used to produce the composition of the present invention are obtained from natural sources. Such chemicals typically include a mixture of chemical species due to their natural origin. Due to the presence of such mixtures, various parameters defined herein can be an average value and may be non-integral.

By the use of the term "hydrocarbyl" in the present specification, it is meant any moiety having the general structure -CₘH₂ₘ₊₁, or a structure based thereon (for example -CₘH₂ₘ) wherein m is an integer between 2 and 36. Unless otherwise stated, the hydrocarbyl moiety may be linear or branched, saturated or unsaturated, unsubstituted or substituted for example with heterogroups such as organic functional groups comprising nitrogen, oxygen or sulphur atoms, and/or interrupted by one or more oxygen, sulphur, silicon atoms, or by silano or dialkylsilcon groups, or mixtures thereof. For the purpose of defining the groups R and R1 in the formula 1 as claimed (definition of the invention) hydrocarbyl is limited to alkyl.

By the use of the term "alkyl" in the present specification, it is meant those hydrocarbyl groups as described above which are unsubstituted and saturated.

By the use of the term "residue" in the present specification, it is meant the part of a reactant molecule which remains in the reaction product compound after a reaction has occurred.

### Compound

In the compound of formula (1), the value of n is preferably in the range from 2 to 10, more preferably 3 to 7, particularly 3, 4 or 5, and especially 5.

The R group is preferably a C₈ to C₂₄, more preferably a C₁₀ to C₂₀, particularly a C₁₂ to C₁₆, and especially a C₁₂ to C₁₄ hydrocarbyl, preferably alkyl, group. R may be linear or branched, but is preferably a branched chain. When the chain is branched, it preferably has not more than two, and more preferably not more than one branch.

The R₁ group is preferably a C₆ to C₂₄, more preferably a C₁₀ to C₂₂, particularly a C₁₄ to C₂₀, and especially a C₁₆ to C₁₈ hydrocarbyl, preferably alkyl, group. R₁ may be linear or branched, but is preferably a linear chain.

In one embodiment, R₁ is branched. When R₁ is branched, it preferably has not more than two branches, and more preferably not more than one branch. When R₁ is branched, the branches are preferably alkyl branches, more preferably methyl, ethyl or propyl branches, more preferably methyl branches, R₁ may comprise a mixture of branch groups. In this case, the predominant branch group is preferably methyl.

R and/or R₁ may be derived from mixtures of materials, in which case the aforementioned carbon chain lengths and/or number of branches refer to the average values by moles or number.

Preferably the compound of the invention is an ester-PPDn-ether compound (otherwise termed an ester-(OCH₂CH₃CH₂)ₙ-ether compound) wherein in formula (1), X is R₁C(O).

R and R₁ in formula (1) may be the same as each other of different. Preferably, R and R₁ in formula (1) are different to each other. In this embodiment, R may contain more carbon atoms than R₁ or fewer carbon atoms than R₁. Preferably, R contains fewer carbon atoms than R₁ in formula (1).

More preferably the compound of the invention is caprylate/caprate-(OCH₂CH₂CH₂)ₙ-isostearyl, wherein in formula (1) X is R₁C(O), with R₁ being C₇₋₁₀, n = 5, and R being an isostearyl group. In this embodiment, R is the residue of isostearyl alcohol and R₁ is the residue of caprylic/capric acid.

The compound may comprise molecules having different values of n, in which case the aforementioned values of n refer to the average values by moles or number, and n may be a non-integer.

The compound of the first aspect of the invention is preferably the reaction product of a polytrimethylene ether glycol, a monohydric alcohol and a monocarboxylic acid.

Preferably, the -(OCH₂CH₂CH₂)ₙ- in formula (1) is the residue of polytrimethylene ether glycol. Preferably, the polytrimethylene ether glycol has between 2 and 15 repeating residual monomer 1,3-propane diol repeating units. Preferably, the 1,3-propane diol monomer residue repeating units are bonded together by ether linkages. The ether linkages are suitably formed by dehydration reaction between neighboring 1,3-propane diol monomers.

Preferably, R in formula (1) is the residue of a monohydric alcohol. The monohydric alcohol is preferably a long-chain primary alcohol, preferably with a chain length of 16 to 26 carbon atoms, which may be derived from natural sources such as fats and oils, Preferably, the alcohol is derived from vegetable sources, although it is also possible to derive the alcohols from animal sources or petrochemical sources. The alcohols used in the present invention will normally be obtained from naturally occurring esters by hydrogenation.

The monohydric alcohol used in the present invention may be a mixture of alcohols of different chain lengths. In this case, the mixture may be a commercially available mixture, or may be made from a combination of different chain length alcohols.

The alcohol used in the present invention preferably contains from 6 to 36 carbon atoms, preferably from 8 to 24 carbon atoms, more preferably from 10 to 20 carbon atoms. The alcohol may be selected from the group comprising capryl alcohol, capric alcohol, lauryl alcohol, myristyl alcohol, cetyl alcohol, cetostearyl alcohol, palmitoleyl alcohol, stearyl alcohol, isostearyl alcohol, oleyl alcohol, arachidyl alcohol, behenyl alcohol, isobehenyl alcohol, erucyl alcohol, lignceryl alcohol, cerotinyl alcohol, montanyl alcohol and melissyl alcohol, Preferably, the alcohol used in the present invention is selected from lauryl alcohol, mysrityl alcohol, cetyl alcohol, cetostearyl alcohol, stearyl alcohol, isostearyl alcohol, arachidyl alcohol, behenyl alcohol, isobehenyl alcohol, lignceryl alcohol and cerotinyl alcohol.

Preferably, R₁ in formula (1) is the residue of a monocarboxylic acid. Preferably, the monocarboxylic acid is a long-chain monocarboxylic acid, preferably with a chain length of 8 to 18 carbon atoms, which may be derived from natural sources such as fats and oils. Preferably, the acid is derived from vegetable sources, although it is also possible to derive the acids from animal sources or petrochemical sources. The acids used in the present invention will normally be obtained from naturally occurring triglycerides.

The monocarboxylic acid used in the present invention may be a mixture of monocarboxylic acids of different chain lengths. In this case, the mixture may be a commercially available mixture, or may be made from a combination of different chain length acids.

The monocarboxylic acid used in the present invention preferably contains from 2 to 36 carbon atoms, preferably from 4 to 24 carbon atoms, more preferably from 6 to 22 carbon atoms and desirably from 8 to 16 carbon atoms. The acid may be selected from the group comprising caproic acid, caprylic acid, capric acid, capric/caprylic acid, lauric acid, myristic acid, palmitic acid, stearic acid, isostearic acid, oleic acid, arachidic acid, behenic acid, isobehenic acid, erucic acid, lignceric acid, cerotinic acid, montanic acid, melissic acid and dimer acid. Preferably, the acid used in the present invention is selected from the group comprising caprylic acid, capric acid, capric/caprylic acid, lauric acid, myristic acid, palmitic acid, stearic acid, and isostearic acid.

### Composition

The invention also provides for a composition comprising a mixture of compounds of the invention as recited above, which can be for example jointly produced.

Preferably the composition comprises a mixture of at least:
(i) the compound(s);

   R₁C(O)-(OCH₂CH₂CH₂)ₙ-OR (1a),

   *i.e*. ester-PPDn-ether compound(s) of the invention, and
(ii) the compound(s):

   R-(OCH₂CH₂CH₂)ₙ-OR, (1b),

   *i.e*. ether-PPDn-ether compound(s) of the invention, wherein
   n, R and R₁ are as recited above.

The composition according to the present invention suitably comprises:
(i) greater than 50% by weight, preferably greater than 60% by weight, more preferably in the range from 65 to 98%, particularly 70 to 95%, and especially 80 to 90% by weight of compound(s) of formula (1a) (ester-PPDn-ether compound),
(ii) less than 30% by weight, preferably less than 20% by weight, more preferably in the range from 1 to 20%, particularly 3 to 15%, and especially 5 to 10% by weight of compound of formula (1b) (ether-PPDn-ether compound), all % given by weight based on the total weight of the composition.

Where R is the residue of a branched alcohol, for example isostearyl alcohol, it will be readily apparent to the skilled person that the compound of formula (1b) will be an alkyl ether-PPDn-alkyl ether compound,

The ratio by weight of compound(s) of formula (1a) to compound(s) of formula (1b) in the composition is suitably greater than 2:1, preferably in the range from 5 to 50:1, more preferably 7 to 25:1, particularly 8 to 15:1, and especially 9 to 11:1.

The composition may also further comprise a di-ester compound of formula (1C):

R₂C(O)-(OCH₂CH₂CH₂)ₘ-OC(O)R₂ (1c),

wherein:
m is an integer from 2 to 15 and
R² is a C₂ to C₃₆ hydrocarbyl group.

The value of m is preferably in the range from 2 to 10, more preferably 3 to 7, particularly 3 to 5, and especially 5. Preferably m is the same as n.

The R₂ group is preferably a C₆ to C₂₄, more preferably a C₁₀ to C₂₂, particularly a C₁₄ to C₂₀, and especially a C₁₆ to C₁₈ hydrocarbyl, preferably alkyl, group. R₂ may be linear or branched, but is preferably a linear chain. When the chain is branched, it preferably has not more than two, and more preferably not more than one branch.

Preferably R₂ is the same as R₁. Preferably R₂ is R₁, which is the residue of a monocarboxylic acid.

The composition according to the present invention suitably comprises less than 30%, preferably less than 20%, more preferably in the range from 0.1 to 15%, particularly 0.5 to 10%, and especially 1 to 7% by weight of compound(s) of formula (1c) (ester-PPDn-ester compound) based on the total weight of the mixture composition.

R, R₁ and/or R₂ may be derived from mixtures of materials, in which case the aforementioned carbon chain lengths and/or number of branches refer to the average values by moles or number.

The composition may further comprise unreacted polytrimethylene ether glycol (PPDn) but suitably the mixture comprises less than 30%, preferably less than 20%, more preferably in the range from 0.1 to 15%, particularly 0.5 to 10%, and especially 1 to 7% by weight of polytrimethylene ether glycol (PPDn) based on the total weight of the mixture composition.

The composition may comprise compounds having molecules with different values of n and/or m, in which case the aforementioned values of n and/or m refer to the average values by moles or number, and n and/or m may be non-integer.

Preferably, the composition comprises compounds having the same value of n and/or m, in the range from 2 to 10, more preferably 3 to 7, particularly 3 to 5, and especially 5.

Preferably, the composition is anhydrous. By the use of the term anhydrous herein, it is meant that the composition preferably comprises a maximum of 10% by weight water. More preferably, the composition comprises a maximum of 7% by weight water, most preferably, 5% and desirably 2% by weight Preferably, the composition comprises 0.01% to 10% by weight water, preferably 0.05% to 5%, most preferably 0.1% to 2% by weight.

The present invention therefore further provides for a composition consisting essentially of
(i) the compound(s):

   R₁C(O)-(OCH₂CH₂CH₂)₂-OR (1a);
(ii) the compound(s):

   R-(OCH₂CH₂CH2)ₙ-OR, (1b);
(iii) the compound(s)

   R₂C(O)-(OCH₂CH₂2CH₂)ₘ-OC(O)R₂ (1c);

   and
(iv) polytrimethylene ether glycol;
wherein
n, R, R₁ and R2 are as recited above.

Preferably, the composition comprises a mixture of compounds which are the reaction products of a polytrimethylene ether glycol, a monohydric alcohol and a monocarboxylic acid.

### Method

The invention also provides for a production method of a compound of the invention or a composition of the invention, the method comprising:
- a first step of preparing an etherified-polytrimethylene ether glycol mixture by reacting polytrimethylene ether glycol and an alcohol having the formula R-OH in the presence of a strong acid catalyst; and
- a second step of esterifying the reaction product obtained in the first step with a fatty carboxylic acid of formula R₁-COOH, in the presence of a strong acid catalyst,
wherein R and R₁ are as recited above.

According to the invention, the strong acid catalyst that may be used may be selected from sulfuric acid, hydrochloric acid, trifluoromethanesulfonic acid, fluorosulfuric acid-antimony pentafluoride 1:1, preferably trifluoromethanesulfonic acid,

More precisely, the first step can be performed at a temperature in the range of 150°C to 220°C for 6 to 24 hours.

More precisely, the second step can be performed at a temperature in the range of 120°C to 180°C for 2 to 12hours.

### Personal Care Product

The invention also provides for a personal care product comprising a compound of the invention or a composition of the invention, as recited above.

The compounds and compositions of the invention may be suitable for use in personal care products including, but not limited to, shampoos, conditioners, conditioning shampoos, body washes, cleansers, hair coloring products and hair dyes, hair relaxers, cosmetics, skin care products, organic sunscreens, inorganic sunscreens, deodorants, antiperspirants, depilatories, skin bronzers, cosmetics removers or the like, lipsticks, foundations, hair mousses, styling gels, anti-aging creams and lotions and anti-acne products. The compounds and compositions of the invention are in particular suitable for use in colour cosmetics, sunscreens, anti-perspirants, anti-aging and moisturizing products.

The present invention provides for the use of a compound of the invention or a composition according to the invention as a rheology modifier in a personal care product. Preferably, the compound or composition of the present invention may increase the viscosity of a personal care product. This may provide a creamier-textured or solidified/semi-solidifled personal care product depending on the concentration of the compound or composition of the present invention included.

The present invention provides for the use of a compound of the invention or a composition according to the invention as a sensory agent in a personal care product. Preferably, the compound or composition of the present invention may improve or alter the skin feel of a personal care product. This may for example provide a less greasy or silkier feeling product depending on the nature of the personal care product.

The present invention provides for the use of a compound of the invention or a composition according to the invention as a pigment dispersing agent and/or a pigment wetter in a personal care product. Preferably, the compound or composition of the present invention may increase the stability of a personal care product, and/or make its production easier, by improving the wetting of pigments and dispersing the solid pigment particles homogeneously in the product to prevent flocculation.

The compound or composition of the present invention is preferably used in a persona! care product at a concentration in the range from 0.1 to 50%, preferably 0.5 to 40%, more preferably 1 to 30%, particularly 2 to 20%, and especially 3 to 10%, by weight of the total composition. The most suitable concentration for a particular personal care product will depend on the specific product and the other ingredients contained therein.

The persona! care product may comprise additional components, for example, additional emollients, carriers, surfactants and the like.

Any additional emollients in the personal care product of the present invention will preferably mainly be an emollient oil of the type used in personal care or cosmetic products. The emollient can and usually will be an oily material which is liquid at ambient temperature. Alternatively it can be solid at ambient temperature, in which case in bulk it will usually be a waxy solid, provided it is liquid at an elevated temperature at which it can be included in and emulsified in the product. The manufacture of the product preferably uses temperatures up to 100°C, more preferably about 80°C, and therefore such solid emollients will preferably have melting temperatures of less than 100°C, and more preferably less than 70°C.

Suitable normally liquid emollient oils include non-polar oils, for example mineral or paraffin, especially isoparaffin, oils, such as that sold by Croda as Arlamol (trade mark) HD; or medium polarity oils, for example vegetable ester oils such as jojoba oil, vegetable glyceride oils, animal glyceride oils, such as that sold by Croda as Crodamol (trade mark) GTCC (caprylic/capric triglyceride), synthetic oils, for example synthetic ester oils, such as isopropyl palmitate and those sold by Croda as Crodamol IPM and Crodamol DOA, ether oils, particularly of two fatty e.g. C8 to C18 alkyl residues, such as that sold by Cognis as Cetiol OE (dicapryl ether), guerbet alcohols such as that sold by Cognis as Eutanol G (octyl dodecanol), or silicone oils, such as dimethicione oil such as those sold by Dow Corning as DC200, cyclomethicone oil, or silicones having polyoxyalkylene side chains to improve their hydrophilicity; or highly polar oils including alkoxylate emollients for example fatty alcohol propoxylates such as that sold by Croda as Arlamol PS15 (propoxylated stearyl alcohol). Suitable emollient materials that can be solid at ambient temperature but liquid at temperatures typically used to make the compositions of this invention include jojoba wax, tallow and coconut wax/oil.

Mixtures of emollients can be used, and in some cases solid emollients may dissolve wholly or partly in liquid emollients or in combination the freezing point of the mixture may be suitably low. Where the emollient composition is a solid (such as fatty alcohols) at ambient temperature, the resulting dispersion may technically not be an emulsion (although in most cases the precise phase of the oily disperse phase cannot readily be determined) but such dispersions behave as if they were true emulsions and the term emulsion is used herein to include such compositions.

In personal care products comprising water, the amount of water present is suitably greater than 5%, preferably in the range from 30 to 90%, more preferably 50 to 90%, particularly 70 to 85%, and especially 75 to 80% by weight of the total composition.

The water phase may include a polyol, e.g. glycerin. In this case, the total water phase including the polyol present in the personal care formulation is suitably greater than 5%, preferably in the range from 30 to 90%, more preferably 50 to 90%, particularly 70 to 85%, and especially 75 to 80% by weight of the total composition.

The personal care products may further comprise cholesterol as an additional moisturisation component. When present, the cholesterol is present at an amount of 0.1 to 5% by weight of the total personal care product.

The personal care product according to the present invention may also contain other additional surfactant materials which form part of the emulsifier system. Other suitable surfactants include relatively hydrophilic surfactants, e.g. having a HLB value of greater than 10, preferably greater than 12, and relatively hydrophobic surfactants e.g. having a HLB value of less than 10, preferably less than 8. Relatively hydrophilic surfactants include alkoxylate surfactants with an average in the range from about 10 to about 100 alkylene oxide, particularly ethylene oxide residues; and relatively hydrophobic surfactants include alkoxylate surfactants preferably with an average in the range from about 3 to about 10 alkylene oxide, particularly ethylene oxide residues.

Personal care or cosmetic products can be divided by viscosity into milks and lotions, which preferably have a low shear viscosity (measured at shear rates of about 0.1 to 10 s⁻¹ as is typically used in Brookfield viscometers) of up to 10,000 mPa.s, and creams which preferably have a low shear viscosity of more than 10,000 mPa.s.

Milks and lotions preferably have a low shear viscosity in the range from 100 to 10,000, more preferably 200 to 5,000, and particularly 300 to 1,000 mPa.s. The amount of the compound or composition according to the present invention present in a milk or lotion is preferably in the range from 2 to 3% by weight of the total composition.

Creams preferably have a low shear viscosity of at least 20,000, more preferably in the range from 30,000 to 80,000, and particularly 40,000 to 70,000 mPa.s, although even higher viscosities e.g. up to about 10⁶ mPa.s, may also be used. The amount of compound or composition according to the present invention present in a cream is preferably in the range from 4 to 5.5% by weight of the total composition.

The personal care products of the invention may be made by conventional emulsification and mixing methods. For example, the compound or composition according to the present invention may be added to (i) the oil phase, which is then added to the aqueous phase, or (ii) both the combined oil and water phases, or (iii) the water phase, which is then added to the oil phase. Method (iii) is preferred. In all of these methods, the resulting mixture can then be emulsified using standard techniques. It is preferred to either heat the aqueous and oil phases usually above about 60°C, e.g. to about 80 to 85°C, or to subject the aqueous phase to high intensity mixing at lower, e.g. about ambient, temperature. Vigorous mixing and the use of moderately elevated temperatures can be combined if desired. The heating and/or high intensity mixing can be carried out before, during or after addition of the oil phase but once emulsified, care should be taken not to destroy the liquid crystal system by excessive mixing or stirring.

The personal care products can also be made by inverse emuisification methods, whereby the compound or composition according to the present invention is added to either the oil phase or the aqueous phase, and the aqueous phase is mixed into the oil phase to initially form a water in oil emulsion. Aqueous phase addition is continued until the system inverts to form an oil in water emulsion. Plainly a substantial amount of aqueous phase will generally be needed to effect inversion and so this method is not likely to be used for high oil phase content emulsions. Vigorous mixing and the use of moderately elevated temperatures can be combined if desired. Heating can be carried out during or after addition of the aqueous phase and before, during or after inversion. High intensity mixing can be carried out during or after addition of the aqueous phase, and before or during inversion

The personal care products may for example be microemulsions or nanoemulsions, having a mean droplet size over a wide range, preferably in the range from 10 to 10,000 nm. In one embodiment, the emulsion droplet size may be reduced, for example by high pressure homogenisation, preferably to a value in the range from 100 to 1,000 nm, more preferably 300 to 600 nm.

The personal care products according to the present invention are preferably stable for greater than one month, more preferably greater than two months, particularly greater than three months, and especially greater than four months at ambient temperature (23°C), and also preferably at 40°C. The stability at even higher temperatures can be particularly important, and therefore the personal care formulation is preferably stable for greater than one week, preferably greater than two weeks, more preferably greater than 3 weeks, particularly greater than one month, and especially greater than two months at 50°C.

Many other components may be included in the personal care product. These components can be oil soluble, water soluble or non-soluble. Examples of such materials include:
(i) preservatives such as those based on parabens (alkyl esters of 4-hydroxybenzoic acid), phenoxyethanol, substituted ureas and hydantoin derivatives e.g. those sold commercially under the trade names Germaben II Nipaguard BPX and Nipaguard DMDMH, when used preferably at a concentration in the range from 0.5 to 2% by weight of the total composition;
(ii) perfumes, when used preferably at a concentration in the range from 0.1 to 10% more preferably up to about 5%, and particularly up to about 2% by weight of the total composition;
(iii) humectants or solvents such as alcohols, polyols such as glycerol and polyethylene glycols, when used preferably at a concentration in the range from 1 to 10% by weight of the total composition;
(iv) sunfilter or sunscreen materials including organic sunscreens and/or inorganic sunscreens including those based on titanium dioxide or zinc oxide; when used preferably at a concentration in the range from 0.1% to 20%, more preferably 1 to 15%, and particularly 2 to 10% by weight of the total composition;
(v) alpha hydroxy acids such as glycolic, citric, lactic, malic, tartaric acids and their esters; self-tanning agents such as dihydroxyacetone, and beta hydroxyl acids such as salicylic acid and their esters;
(vi) anti-aging, cell-turnover-improving, and antimicrobial, particularly anti-acne, components such as salicylic acid;
(vii) vitamins and their precursors including: (a) Vitamin A, e.g. as retinyl palmitate and other tretinoin precursor molecules, (b) Vitamin B, e.g. as panthenol and its derivatives, (c) Vitamin C, e.g. as ascorbic acid and its derivatives, (d) Vitamin E, e.g. as tocopheryl acetate, (e) Vitamin F, e.g. as polyunsaturated fatty acid esters such as gamma-linolenic acid esters;
(viii) skin care agents such as ceramides either as natural materials or functional mimics of natural ceramides;
(ix) phospholipids, such as synthetic phospholipids or natural phospholipids, e.g. lecithin;
(x) vesicle-containing formulations;
(xi) botanical extracts with beneficial skin care properties;
(xii) skin whiteners such as ODA White, Mediatone (trade marks) sold by Sederma, a member of Croda International Pic kojic acid, arbutin and similar materials;
(xiii) skin repair compounds actives such as Allantoin and similar series;
(xiv) caffeine and similar compounds;
(xv) cooling additives such as menthol or camphor;
(xvi) insect repellents such as N,N-diethyl-3-methylbenzamide (DEET) and citrus or eucalyptus oils;
(xvii) essential oils;
(xviii) ethanol; and
(xix) pigments, including microfine pigments, particularly oxides and silicates, e.g. iron oxide, particularly coated iron oxides, and/or titanium dioxide, and ceramic materials such as boron nitride, or other solid components, such as are used in makeup and cosmetics, to give suspoemulsions, preferably used in an amount in the range from 1 to 15%, more preferably at least 5% and particularly approximately 10%.

The compound, composition and personal care product according to the present invention are suitable for use in a wide range of compositions and end-use applications, such as moisturizers, sunscreens, after sun products, body butters, gel creams, high perfume containing products, perfume creams, baby care products, hair conditioners, skin toning and skin whitening products, water-free products, anti-perspirants and deodorant products, tanning products, cleansers, 2-in-1 foaming emulsions, multiple emulsions, preservative free products, emulsifier free products, mild formulations, scrub formulations e.g. containing solid beads, silicone in water formulations, pigment containing products, sprayable emulsions, colour cosmetics, conditioners, shower products, foaming emulsions, make-up remover, eye make-up remover, and wipes.

Personal care products containing a compound or a composition according to the present invention may have a pH value over a wide range, preferably in the range from 3 to 13, more preferably 4 to 9, and especially 5 to 6.

Any of the above features may be taken in any combination and with any aspect of the invention,

### Examples

The invention is illustrated by the following non-limiting examples. All parts and percentages are given by weight unless otherwise stated.

It will be understood that all tests and physical properties listed have been determined at atmospheric pressure and ambient temperature (i.e. about 23°C), unless otherwise stated herein, or unless otherwise stated in the referenced test methods and procedures.

### Test Methods

In this specification the following test methods have been used:
(i) Emulsion stability was assessed by observing the emulsions after storage for 3 months at ambient temperature (23°C), cold at 5°C or under elevated temperature storage at 40°C, 45°C and 50°C. Measuring storage stability at 50°C is a severe test. The emulsions were also assessed for their freeze-thaw stability using a cycling oven (-10°C to 40°C in 24 hours). The composition was stable if no visible separation of the emulsion occurred. The stability of the emulsions was also assessed by monitoring the size of the dispersed phase water particles over a three month period. The particle size was measured using a Malvern Mastersizer 2000 that measures the size of the dispersed phase particles using laser diffraction.
(ii) Emulsion viscosity was measured at 23°C with a Brookfield LVT viscometer using an appropriate spindle (LV1, LV2, LV3, or LV4) depending on the viscosity of the emulsion. The emulsion was tested at 10 rpm (0.1 Hz), 1 day after making the emulsion and results are quoted in mPa.s.
(iii) The hydroxyl value is defined as the number of mg of potassium hydroxide equivalent to the hydroxyl content of 1 g of sample, and was measured by acetylation followed by hydrolysation of excess acetic anhydride. The acetic acid formed was subsequently titrated with an ethanolic potassium hydroxide solution.
(iv) The acid value is defined as the number of mg of potassium hydroxide required to neutralize the free acids in 1 g of sample, and was measured by direct titration with a standard potassium hydroxide solution,
(v) The particle size was determined according to ASTM method D1210 - 05(2014) Standard Test Method for Fineness of Dispersion of Pigment-Vehicle Systems by Hegman-Type Guage.

Where mentioned herein, concentrations are given by weight based on the total weight of the compound / composition / formulation.

### Preparation Examples

### Example 1: General method of preparation of a mixture composition of the invention comprising the R₁(O)-(OCH₂CH₂CH₂)ₙ-OR compound of the invention.

### First step of preparation of a precursor mixture composition comprising the R-(OCH₂CH₂CH₂)ₙ-OH compound.

3-10 moles of vegetable derived 1,3-propanediol, 1 mole of fatty alcohol of formula R-OH and concentrated sulfuric acid catalyst were added to a reaction flask with nitrogen inlet, mechanical stirrer and a distillation head. The mixture was heated to 150-170°C with a nitrogen sparge and held for 2.5 hours. The temperature was then increased to 175-250°C and held for up to 24 hours. At this point, the temperature was lowered to 75°C and a 45% KOH solution and de-ionized water were added. The resulting aqueous mixture was held at 75°C and stirred for 2-4 hours under nitrogen to hydrolyze the acid ester formed during the acid catalyzed polycondensation and to neutralize the residual acid. The reaction mixture was then allowed to stand for phase-separation with the bottom layer being the aqueous phase and the top layer being the organic phase. After the aqueous phase was removed, the organic phase was dried under vacuum and filtered.

This step resulted in the preparation of a mixture composition comprising mostly the monocapped ether compound H-(OCH₂CH₂CH₂)ₙ-OR (in weight with regard to the total weight of the composition).

The composition obtained also comprises in minor amount the dicapped compound R-(OCH₂CH₂CH₂)ₙ-OR,

### Second step of esterification of the precursor mixture composition

1 mole of the product mixture prepared in the first step, 1 mole of fatty acid of formula R¹COOH and 0.1 w/w% of tin II oxalate catalyst were added to a clean, dry flask with nitrogen inlet, mechanical stirrer and a distillation head. The reaction mixture was heated to and held at between 180-220°C while being tracked by acid value drop. Once an acid value of <2 was achieved, the batch was cooled to 90°C, dried and filtered .

This step resulted in the preparation of a composition mostly comprising the monocapped compound of the invention R¹C(O)-(OCH₂CH₂CH₂)ₙ-OR and in minor amounts the dicapped compound of the invention R-(OCH₂CH₂CH₂)ₙ-OR formed in the first step, and the diester compound R¹C(O)-(OCH₂CH₂CH₂)ₙ-O(CO)R¹.

No residual polypropane diol or free alcohol was detected.

### Preparation of isostearate-PPDS-istearyl according to this method

First step: the procedure was conducted using 894 g of vegetable derived 1,3-propanediol, 657 g of isostearyl alcohol and 4.7 g concentrated sulfuric acid were added to a 3L four-neck round bottom flask. After the reaction, 4.4 g of a 45% KOH solution and 320 g of de-ionized water were added and the resulting aqueous mixture was held at 75°C and stirred for 4 hours under nitrogen.

Second step: 439 g of the product PPD-5 isostearyl alcohol prepared in the first step, 237 g of isostearic acid and 0.8 g tin II oxalate catalyst were added to a clean, dry 1 L four-neck round bottom flask with nitrogen inlet, mechanical stirrer and a distillation head. The reaction mixture was heated to and held at between 180-220°C while being tracked by acid value drop.

### Example 2: Distribution

The following table 1 outlines results of NMR data used to determine distribution for different products prepared according to the above mentioned first step of the method with different length PPD chains and fatty alcohol used for capping selected from Cetearyl alcohol (sold under the tradename Crodacol 1618 supplied by Croda Inc., comprising a distribution of cetyl (C16) and stearyl (C18) alcohols) and myristyl alcohol (sold under the tradename Crodacol M95 supplied by Croda Inc., consisting of C14 alcohol).

**Table1:**

| | monocaped ether compound % H-(OCH₂CH₂CH₂)ₙ-OR (n repeating units) | dicaped ether compound % R-(OCH₂CH₂CH₂)ₙ-OR (n repeating units) | PPD % (n repeating units) | Free Alcohol |
|---|---|---|---|---|
| PPD3-Myristyl | 52.4 (~3) | 10.9 (~1) | 27 (~7) | 9.7 |
| PPD3-Myristyl | 66.7 (~3) | 15.7 (~1) | 3.5 (~7) | 14.1 |
| PPD3-Cetearyl | 60.6 (~3) | 14.4 (~3) | 6.2 (~3) | 18.9 |
| PPD5-Myristyl | 61.2 (~4) | 7.5 (~1) | 23.6 (~7) | 7.8 |
| PPD5-Myristyl | 75.0 (~5) | 11.3 (~5) | 8.2 (~5) | 5.6 |
| PPD5-Cetearyl | 71.1 (~5) | 11.3 (~5) | 9.0 (~5) | 8.7 |
| PPD10-Cetearyl | 56.9 (~10) | 7.8 (~10) | 31.6 (~10) | 3.6 |

The method of example 1 was applied to prepare a mixture composition comprising the cetearyl-PPD5-caprylate/caprate compound of the invention (from a mixture of caprylic (C8) and capric (C10) acids reacted with the PPD5-cetearyl alcohol mixture mentioned above) - the distribution of the different compounds of the mixture was determined by NMR analysis (Standard 1H and 13C NMR experiments) and is shown in the following table 2.

**Table 2:**

| Compound | Weight % / total weight of the composition |
|---|---|
| caprylate/caprate-PPD5-cetearyl | 81.15 |
| cetearyl-PPD5-cetearyl | 10.54 |
| caprylate/caprate-PPD 5-caprylate/caprate | 8.31 |

### Application Examples

In the following examples, the mixture composition of the invention is used which comprises the ester-PPDn-ether compound as the major component. However, for simplification, the name "ester-PPDn-ether" is used in the text to designate the mixture.

### Example 3: Evaluation of caprate/caprylate-PPD5-isostearyl in color cosmetic products of the dispersing properties of inorganic solid pigments/dyes.

The objective was to evaluate the pigment wetting capacity of the caprate/caprylate-PPD5-isostearyl of the invention compared to most commonly used dispersion emollient media in colour cosmetics namely:
- Cromollient^{™} DP3A, a di-ester of propoxylated myristyl alcohol and adipic acid, marketed by Croda (INCI name: DI-PPG-3 Myristyl Ether Adipate);
- Crodamol^{™} GTCC, a caprate/caprylate triglyceride (INCI name: Caprylic/Capric Triglyceride); and
- Castor oil (INCI name: Ricinus Communis (Castor) Seed Oil).

A red pigment (Red 7 Calcium Lake - INCI name: Cl 15850) at a ratio media/pigment of 55:45 was tested. Each emollient/media was mixed with this pigment and the dispersion formed was mixed with an overhead stirrer for 1/2hour, followed by three passes through a three roll mill.

### Viscosity test:

Measurements of viscosity after stabilization (after 24hours) of the dispersions were taken with a Brookfield viscometer and are reported in the following table 3.

**Table 3: Viscosity in cps of the dispersions**

| **Emollient** | **Viscosity (cps)** |
|---|---|
| Caprate/caprylate-PPD5-isostearyl of the invention | 30,000 |
| Cromollient^{™} DP3A | 25,000 |
| Crodamol^{™} GTCC | 5,000 |
| Castor oil | 250,000 |

### Particle size test:

The standardized test of Hegman Gauge and Fineness of Grind was used to approximate the particle size. It consists of placing a small volume of product on the deep end and drawing it with a straight scraper toward the shallow end. The position on the scale where oversize particle and their tracks appear is rated for determination of dispersion. The results are reported in the following table 4.

**Table 4: Particle size approximation in µm in the dispersions**

| **Emollient** | **Hegman Gauge** |
|---|---|
| Caprate/caprylate-PPD5-isostearyl of the invention | 4.25 µm |
| Cromollient^{™} DP3A | 7.00 µm |
| Crodamol^{™} GTCC | 11.00 µm |
| Castor oil | 5.75 µm |

Lower viscosity is not directly proportional to better pigment dispersion. This is shown by the results above for the Crodamol GTCC exhibiting the lowest viscosity (5,000cps) but the highest particle size (11.00µm) in the dispersion.

In fact, dispersion mixture needs to be optimal and requires that pigmentary particle size is significantly reduced to prevent any agglomeration or re-agglomeration. A typical pigment dispersion particle size is 5 µm. Below 5µm depicts superiority in the dispersion. A synergy between low particle size dispersion and a satisfactory viscosity is more favorable than a low viscosity evaluation alone.

The caprate/caprylate-PPD5-isostearyl of the invention exhibits a particle size below 5 µm together with a satisfactory viscosity level comparable to conventional used emollient like the Cromollient^{™}DP3A.

### Color intensity via Colorimetry

The color intensity was performed by evaluating the L^{∗} and a^{∗} values of each dispersion.

Defined by the "Commission Internationale de l'Eclairage" (CIE), the L^{∗}a^{∗}b^{∗} color space was modeled after a color-opponent theory stating that two colors cannot be red and green at the same time or yellow and blue at the same time.

L^{∗} indicates lightness/darkness of the sample.

a^{∗} is the red/green coordinate.

b^{∗} is the yellow/blue coordinate.

The following table 6 shows the results.

**Table 6:**

| **Emollient** | **L^{∗}** | **a^{∗} b^{∗} c^{∗} h^{∗}** | | | |
|---|---|---|---|---|---|
| Caprate/caprylate-PPD5-isostearyl of the invention | 32.35 | 33.60 | 15.68 | 37.10 | 25.10 |
| Cromollient^{™} DP3A | 32.45 | 32,13 | 15.58 | 35.71 | 25.87 |
| Crodamol^{™} GTCC | 32.45 | 35.33 | 16.96 | 39.19 | 25.64 |
| Castor oil | 31.30 | 36.78 | 12.78 | 38.97 | 19.31 |

Cromollient DP3A and Crodamol GTCC exhibit the lightest sample tested and the highest red coordinate belongs to castor oil. Overall, the lightness result of the compound of the invention (32.35) is only 0.10% different than its counterparts Cromollient DP3A and Crodamol GTCC (32.45), and the redness/red color intensity for the caprate/caprylate-PPD5-isostearyl of the invention (33.60) is 8.00% different than the emollient that exhibits the highest color vibrancy. It can be concluded, therefore, that the color intensity of pigment dispersions with the compound of the invention is comparable to conventional used emollients.

### Example 4: Personal care or cosmetic products according to the invention

The compounds of the invention have very good compatibility with many different types of skin care systems ranging from sticks, waxes, pigments, lotions, sunscreens, with excellent stability as for example shown by the following test.

### Stability test:

A wax system was made for a simple lip balm chassis. The system consisted of waxes and super sterol ester. Two systems were made, one with the Cromollient^{™} DP3A, and another with the caprate/caprylate-PPD5-isostearyl of the invention using 12% of each. Both were placed in 50°C oven for 24hours. The lip balm with the Cromollient^{™} DP3A experienced significant sweating while the system with the compound of the invention was completely intact and unchanged.

Examples of other personal care and cosmetic products according to the invention are given below.

As shown in Tables 7-15 below, weights are collective for all Parts. For example, as shown in Table 7, the sum of the weights in Parts A-C is 100%.

The ester-PPDn-ether compound of the invention mentioned in these formulations is present either alone or as part of a mixture composition according to the invention comprising the named compound as the major component (w/w) compared to the corresponding ether-PPDn-ether and ester-PPDn-ester components.

**Table 7: Sprayable Sunscreen Lotion**

| **Ingredients** | **Weight %** |
|---|---|
| **Part A:** | |
| Deionized Water | 61.20 |
| Xanthan Gum | 0.20 |
| CRODAFOS CS-20 ACID (Cetearyl Alcohol, Ceteth-20 Phosphate, Dicetyl Phosphate) | 4.00 |
| Glycerin | 5.00 |
| Triethanplamine (TEA, 98%) | 0.10 |

| **Part B:** | |
|---|---|
| CRODAMOL AB (C12-C15 Alkyl Benzoate) | 3.00 |
| Caprate/caprylate-PPD5-isostearyl of the invention | 5.00 |
| Octinoate | 7.50 |
| Octisalate | 5.00 |
| Oxybenzone | 5.00 |
| CHROMAVEIL (Cas# 1030827-59-8) | 3.00 |

| **Part C:** | |
|---|---|
| Propylene Glycol, Diazolidinyl Urea, Methyl Paraben, Propyl Paraben | 1.00 |

The sprayable sunscreen disclosed in Table 7 was formed by heating deionized water to a temperature between about 75°C to about 80°C, then adding the xanthan gum, and allowing the xanthan gum to completely hydrate. The remaining ingredients of Part A in Table 7 were added one at a time, each ingredient being allowed to fully dissolve before adding the next ingredient, Separately, Part B was prepared by combining the ingredients of Part B in Table 7 and heating to about 75°C. Once Part A and Part B were prepared, Parts A and B were combined and maintained at a temperature between about 75°C to about 80°C for about 15 minutes. The mixture of Parts A and B was then cooled to about 45°C. Once cooled, Part C was added to the mixture of Parts A and B. The combined mixture was then be cooled and packaged.

**Table 8: Acid cream**

| **Ingredients** | **Weight %** |
|---|---|
| **Part A:** | |
| CRODAFOS CS-20 ACID (Cetearyl Alcohol, Ceteth-20 Phosphate, Dicetyl Phosphate) | 8.0 |
| CRODACOL C-70 (Cetyl Alcohol) | 2.0 |
| Mineral Oil | 14.50 |
| CRODAMOL STS (PPG-3 Benzyl Myristate) | 3.00 |
| SUPER STEROL ESTER (C10-C30 Cholesterol/Lanosterol Esters) | 5.00 |
| Ethylhexylmethoxy Cinnamate | 5.00 |

| **Part B:** | |
|---|---|
| Salicylic Acid | 2.00 |
| **Caprate/Caprilate-PPD5-isostearyl of the invention** | 3.00 |

| **Part C**: | |
|---|---|
| Deionized water | 52.20 |
| Glycerin | 5.00 |
| Sodium Hydroxide (pellets, 97%+) | 0.30 |

The acid cream disclosed in Table 8 was formed by combining and heating the components of Part A to about 80°C. Part B added to Part A, and the acid crystals were allowed to dissolve. Separately, the components of Part C were combined and heated to about 80°C. Then, Part C was added to the existing mixture of Parts A and B while mixing. The combined mixture of Parts A, B and C were maintained at about 80°C for about 20 minutes, and then cooled to room temperature without using a water bath.

**Table 9: Microemulsion Styling Gel**

| **Ingredients** | **Weight %** |
|---|---|
| **Part A:** | |
| INCROCAS 30 (PEG-30 Castor Oil) | 10.00 |
| Mineral Oil | 10.00 |
| Brij O10 (Oleth-10) | 10.00 |
| Brij O20 (Oleth-20) | 11.00 |
| INCROQUAT BEHENYL 18-MEA (Behentrimonium Methosulfate, Quaternium-33, Cetearyl Alcohol) | 2.00 |
| Propyl Paraben | 0.20 |

| **Part B:** | |
|---|---|
| Deionized water | 40.60 |
| Glycerin | 7.00 |
| Polypropylene Glycol | 5.00 |
| **Behenate-PPD4-isostearyl of the invention** | 4.00 |
| Methyl Paraben | 0.20 |

The styling gel disclosed in Table 9 was formed by combining the ingredients of Part A while mixing and heating at a temperature between about 90°C to about 95°C. Separately, the ingredients of Part B were combined while mixing and heating at a temperature between about 90°C to about 95°C. Then, Part B was added to Part A while rapidly mixing and maintaining the temperature for about 15 minutes. Then, the mixture of Parts A and B was cooled to about 60°C and poured into molds before the mixture reached a set point of about 50 to about 55°C, pH: 6.00 ±0.50.

**Table 10: Cationic Skin Moisturizing Lotion**

| **Ingredients** | **Weight %** |
|---|---|
| **Part A:** | |
| Deionized water | 76.98 |
| **Laurate-PPD5-behenyl of the invention** | 10.00 |
| Glycerin | 5.00 |
| CRODACOL S-70 (Stearyl Alcohol) | 2.52 |
| INCROQUAT BEHENYL TMS-50 (Behentrimonium Methosulfate, Butylene Glycol, Cetearyl Alcohol) | 2.50 |

| **Part B:** | |
|---|---|
| Cyclomethicone | 2.00 |
| Polypropylene Glycol, Diazolidinyl Urea, Methyl Paraben, Propyl Paraben | 1.00 |

The moisturizing lotion disclosed in Table 10 was formed by combining the ingredients of Part A while mixing and heating at a temperature between about 75°C to about 80°C. The heating temperature of Part A was maintained for about 15 minutes, and then the mixture of Part A was cooled to about 50°C. The ingredients of Part B were then added individually to Part A while mixing. The lotion was then be cooled and packaged.

**Table 11; Conditioning Shampoo with UV Protection**

| **Ingredients** | **Weight %** |
|---|---|
| **Part A:** | |
| Deionized Water | 29.22 |
| Glycerin | 1.00 |
| Disodium EDTA | 0.20 |
| PEG 7M | 0.20 |

| **Part B:** | |
|---|---|
| Ammonium Lauryl Sulfate (ALS), 28% | 14.00 |
| Ammonium Lauryl Ether Sulfate (ALES), 25% | 40.00 |
| INCRONAM 30 (Cocamidopropyl Betaine) | 6.60 |
| LUSTREPLEX (Polyquaterium-70 (and) Dipropylene Glycol) | 1.42 |
| Glycol Distearate | 0.80 |
| CHROMAVEIL (cas# 1030827-59-8) | 2.50 |
| Silicone | 0.50 |
| **Caprate/Caprilate-PPD5-isostearyl of the invention** | 2.00 |

| **Part C:** | |
|---|---|
| CRODASONE W (Hydrolyzed Wheat Protein PG-Propyl Silanetriol) | 1.00 |
| CROSILK LIQUID (Silk Amino Acids) | 0.20 |
| Methylisothiazolinone | 0.10 |

| **Part D:** | |
|---|---|
| CROTHIX LIQUID (PEG-150 Pentaerythrityl Tetrastearate (and) PEG-6 Caprylic/Capric Glycerides (and) Water) | 0.26 |

The conditioning shampoo disclosed in Table 11 was formed by combining the initial three ingredients of Part A and mixing until the solid had dissolved. Then, the remaining ingredient, PEG 7M was added and mixed until hydrated. Then, the ingredients of Part B were added to the mixture of Part A at temperatures up to about 75°C and mixed until solids had dissolved. The Part A/Part B mixture was cooled to about 40°C, and then the ingredients of Part C were added. Once the ingredients of Part C had been dissolved in the Part A/Part B mixture, the pH of the combined Part A/Part 8/Part C mixture was be determined. If the pH was outside a range of about 5.5 to about 7.0, the pH was adjusted to be within that range. Then, the ingredients of Part D were added while slowly mixing between about 100 to about 300 rotations per minute (rpm).

**Table 12: Hair Conditioning Rinse with UV Protection**

| **Ingredients** | **Weight %** |
|---|---|
| **Part A:** | |
| Crodazozosoft DBQ (Quaternium 91 (and) Cetrimonium Methosulfate (and) Cetearyl Alcohol | 2.50 |
| Dimethicone | 5.00 |
| CRODACOL S-70 (Stearyl Alcohol) | 2.50 |
| Wheat Germ Oil | 1.00 |
| BHT | 0.10 |
| CHROMAVEIL (cas# 1030827-59-8) | 2.00 |
| **Isostearate-PPDS-isostearyl of the invention** | 2.00 |

| **Part B:** | |
|---|---|
| Deionized Water | 80.90 |

| **Part C:** | |
|---|---|
| Propylene Glycol (and) Diazolidinyl Urea (and) Methylparaben (and) Propylparaben | 1.00 |

| **Part D:** | |
|---|---|
| HYDROTRITICUM WAA (Wheat Amino Acids) | 3.00 |

The hair conditioning rinse disclosed in Table 12 was formed by combining the ingredients of Part A while mixing and heating at a temperature between about 80°C to about 85°C. Separately, the ingredients of Part B were heated at a temperature between about 80°C to about 85°C. Then, Part B was added to Part A while mixing and maintaining the temperature for about 15 minutes. Then, the mixture of Parts A and B was cooled to about 50°C. After cooling, the ingredients of Part C were added while mixing, followed by the ingredients of Part D.

**Table 13: Anti-Perspirant**

| **Ingredients** | **Weight %** |
|---|---|
| **Part A:** | |
| **Behenate-PPD3-behenyl of the invention** | 47.00 |
| ARLAMOL^{™} PM3 (PPG-3 Myristyl Ether) | 3.00 |
| ARLAMOL PB14 (PPG-14 Butyl Ether) | 2.00 |
| ARLAMOL PC10 (PPG-10 Cetyl Ether) | 2.00 |
| BRIJ^{™} S10 (Steareth-10) | 1.00 |

| **Part B:** | |
|---|---|
| CRODACOL^{™} S95 (Stearyl Alcohol) | 16.00 |

| **Ingredients** | **Weight %** |
|---|---|
| Hydrogenated Castor Oil | 3.50 |
| Corn Starch Modified | 3.00 |
| Fumed Silica | 0.50 |

| **Part C:** | |
|---|---|
| Aluminum Zirconium Tetrachlorohydrex GLY | 22.00 |

The anti-perspirant disclosed in Table 13 was formed by combining the ingredients of Part A while mixing and heating at a temperature are then added to the mixture. Once the first two ingredients of Part A were dissolved, then the remaining ingredients of Part B were added. Each remaining ingredient of Part B was added individually. The mixture of Parts A and B was mixed until it was homogeneous. Then, the mixture is cooled to a temperature between about 60°C to about 65°C, While maintaining that temperature, the ingredients of Part C were added while mixing and the mixture was mixed until it was homogeneous. Then, the mixture is cooled to a temperature of about 50°C, and poured into molds to form a solid anti-perspirant.

**Table 14: Hair Color Crème**

| **Ingredients** | **Weight %** |
|---|---|
| **Part A:** | |
| Deionized Water | 58.65 |
| Sodium Sulfite | 0.30 |
| EDTA | 0.20 |
| Sodium Isoascorbate | 0.30 |
| IncromidE^{™} CDEA (Cocamide DEA) | 3.00 |
| Glycerin | 0.50 |
| Incromectant^{™} AMEA 100 (Acetamide MEA) | 0.50 |
| p-Phenylenediamine | 0.05 |
| 4-amino-2-hydroxytoluene | 0.80 |
| Resorcinol | 0.20 |
| 1-Napthol | 0.20 |
| p-aminophenol | 0.80 |

| **Part B:** | |
|---|---|
| KeraTint EZ (Cetyl Alcohol (and) Stearyl Alcohol (and) PPG-5 Ceteth-20 & Dicetyl Phosphate (and) Ceteth-10 Phosphate (and) Behentrimonium Methosulfate) | 19.00 |
| **Oleate-PPD5-isostearyl of the invention** | 2.00 |

| **Part C:** | |
|---|---|
| CRODASONE W (Hydrolyzed Wheat Protein PG-Propyl Silanetriol) | 2.00 |
| CROSILKQUAT (Cocamidopropyl Hydroxypropyl Silk Amino Acids) | 0.50 |
| CRODATERIC^{™} CAS 50 (Cocamidopropyl Hydroxysultaine) | 1.00 |

| **Part D**: | |
|---|---|
| Ammonia | 10.00 |

The hair color creme disclosed in Table 14 was formed by combining the ingredients of Part A while mixing and heating to a temperature of up to about 70°C, and until the ingredients were dissolved. Separately, the ingredients of Part B were heated up to about 70°C. Then, Part B was added to Part A while mixing and maintaining the temperature for about 15 minutes. Then, the mixture of Parts A and B were cooled to at temperature of about 45°C. After cooling, the ingredients of Part C were added while mixing. After further cooling or at a temperature of about 35°C, the ingredients of Part D were added to Parts A/B/C and the mixture mixed for about 1 hour. The mixture may then be mixed with a hydrogen peroxide developer at a weight ratio of about 1:1, at the time of application to hair.

**Table 15: Lip Stick**

| **Ingredients** | **Weight %** |
|---|---|
| **Part A:** | |
| Syncrowax OSW (Tribehenin (and) Sorbitol/Sebacic Acid Coploymer Behenate) | 3.50 |
| Castor Oil | 23.55 |
| Crodamol PTIS (Pentaerythrityl Tetraisostearate) | 16.50 |
| **Isostearate-PPD5-cetearyl of the invention** | 11.00 |
| Super Sterol Ester (C10-30 Cholesterol/Lanosterol Esters) | 5.00 |
| Microcrystalline Wax | 2.00 |
| Ozokerite | 1.50 |
| Carnauba Wax | 1.25 |
| Candelilla Wax (refined) | 5.00 |

| **Part B:** | |
|---|---|
| Castor Oil | 18.20 |
| C19-7711 Red 7 Lake (Sun Chemical Corp.) | 1.50 |
| C19-7712 Red 6 Lake (Sun Chemical Corp.) | 3.00 |
| C39-4433 Blue 1 Lake(Sun Chemical Corp.) | 0.95 |
| C33-8073 Cosmetic Yellow (Sun Chemical Corp.) | 1.00 |

| **Part C:** | |
|---|---|
| Mica (Mearlmica MMCF) | 4.00 |
| Titanium Dioxide (and) Mica (and) Silica (Mearlmica MMCF) | 2.00 |
| Acorbyl Palmitate | 0.05 |

The lip stick disclosed in Table 15 was formed by combining the ingredients of Part A while mixing and heating between a temperature of about 85°C to about 90°C, and until the mixture was clear. Separately, ingredients of Part B were mixed until particle size was less than about 25 microns. In some embodiments, a three-roll mill was utilized to achieve the desired particle size. Then, Part B was added to Part A while continuing to mix until the mixture of Parts A and B was homogeneous. Then, the mixture of Parts A and B was cooled to about 75°C, After cooling, the ingredients of Part C were added while mixing and then the mixture was poured into molds at a temperature of about 70°C.

**Table 16: Make-Up Remover**

| **Ingredients** | **Weight %** |
|---|---|
| **Part A:** | |
| Deionized Water | 63.0 |
| Propylene Glycol | 5.0 |
| | |

| **Part B:** | |
|---|---|
| **Caprate/Caprilate-PPD5-isostearyl of the invention** | 25.00 |
| Incroquat TMS -50 (Behentrimonium Methosulfate (and) Cetyl Alcohol (and) Butylene Glycol) | 4.00 |
| Crodacol C-70 (Cetyl Alcohol) | 2.00 |
| | |

| **Part C** | |
|---|---|
| Propylene Glycol (and) Diazolidinyl Urea (and) Methylparaben (and) Propylparaben | 1.00 |

The Make-up remover disclosed in Table 16 was formed by combining the ingredients of Part A while mixing and heating to a temperature of about 80°C. Separately, ingredients of Part B were mixed and heated to a temperature of about 80°C, Part B was then added to Part A while continuing to mix and maintain heat at about 80C for 10 minutes. The mixture of Parts A and B was cooled to about 40°C, and then Part C was added and the resulting formulation cooled to room temperature.

## Claims

1. A compound of the following formula 1:
X-(OCH₂CH₂CH₂)ₙ-OR (1)
wherein:
n is an integer from 2 to 15,
R is a C₆ to C₃₆ hydrocarbyl group,
X is R or R₁C(O),
R₁ is a C₂ to C₃₆ hydrocarbyl group, and
wherein R and R₁ groups are alkyl groups.

2. The compound according to claim 1, wherein the R group is a C₁₀ to C₂₀ hydrocarbyl group.

3. The compound or composition according to claim 1 or 2, wherein the R₁ group is a C₁₀ to C₂₂ hydrocarbyl group.

4. The compound according to any one of claims 1 to 3, wherein the value of n is in the range from 3 to 7.

5. The compound according to any one of claims 1 to 4, being the caprylate/caprate--(OCH₂CH₂CH₂)₅--isostearyl, wherein in formula (1) X is R₁C(O) with R₁ being C₇₋₁₀, n is 5, and R is an isostearyl group.

6. A composition comprising a mixture of compounds of any one of claims 1 to 5.

7. A composition according to claim 6, wherein the mixture comprises at least:
(i) the compound(s):
R-(OCH₂CH₂CH₂)ₙ-OR, (1a) ;
and
(ii) the compound(s):
R₁C(O)-(OCH₂CH₂CH₂)ₙ-OR (1b).

8. The composition according to claim 6 or 7, wherein the ratio by weight, based on the total weight of the composition, between compound(s) of formula (1a) and compound(s) of formula (1b) is greater than 2:1.

9. The composition according to any one of claims 6 to 8, further comprising at least a compound of formula (1c):
R₁C(O)-(OCH₂CH₂CH₂)ₘ-OR₂CO (1c),
wherein:
m is an integer from 2 to 15,
R₁ and R₂ are independently a C₂ to C₃₆ hydrocarbyl group.

10. The composition according to claim 9, wherein the R₁ and R₂ groups are C₁₀ to C₂₂ alkyl groups.

11. The composition according to claim 10, wherein the R₁ and R₂ are identical groups.

12. The composition according to any one of claim 9 to 11, wherein the value of m is in the range from 3 to 7.

13. The composition according to any one of claim 9 to 12, wherein the value of n and m are identical.

14. The composition according to any one of claims 9 to 13, comprising less than 30% by weight of compound(s) of formula (1c) with regard to the total weight of the mixture composition.

15. The composition according to any one of claims 6 to 14 comprising
(i) greater than 50% by weight of compound(s) of formula (1a):
R₁CO-(OCH₂CH₂CH₂)ₙ-OR,
and
(ii) less than 30% by weight of compound(s) of formula (1b):
R-(OCH₂CH₂CH₂)ₙ-OR,
all % by weight based on the total weight of the composition.

16. A personal care product comprising the compound according to any one of claims 1 to 5 or the composition according to any one of claims 6 to 15.

17. The personal care product of claim 16 wherein the product comprises the compound according to any one of claims 1 to 5 or the composition according to any one of claims 6 to 15 as a rheology modifier.

18. The personal care product of claim 16 or claim 17, wherein the personal care product further comprises a personal care and/or cosmetic active ingredient comprising an active agent selected from a sunscreen agent, an anti-perspirant agent, an anti-aging agent, a moisturizing agent, a hair or scalp care agent, and a coloring or pigment agent.

19. Use of the compound according to any one of claims 1 to 5, or of the composition according to any one of claims 6 to 15, as a sensory agent and/or pigment wetting agent in a cosmetic or personal care product.

20. A method of producing a compound according to any one of claims 1 to 5 or of a composition according to any one claims 6 to 15, the method comprising:
∘ a first step of preparing an etherified-polytrimethylene ether glycol mixture by reacting polytrimethylene ether glycol and an alcohol having the formula R-OH in the presence of a strong acid catalyst; and
∘ a second step of esterifying the reaction product obtained in the first step with a fatty carboxylic acid of formula R₁-COOH, in the presence of a strong acid catalyst.

## Patentansprüche

1. Verbindung der folgenden Formel 1:
X-(OCH₂CH₂CH₂)ₙ-OR (1),
wobei:
n für eine ganze Zahl von 2 bis 15 steht,
R für eine C₆- bis C₃₆-Hydrocarbylgruppe steht,
X für R oder R₁C(O) steht,
R₁ für eine C₂- bis C₃₆-Hydrocarbylgruppe steht und
wobei die Gruppen R und R₁ für Alkylgruppen stehen.

2. Verbindung nach Anspruch 1, wobei die Gruppe R für eine C₁₀- bis C₂₀-Hydrocarbylgruppe steht.

3. Verbindung oder Zusammensetzung nach Anspruch 1 oder 2, wobei die Gruppe R₁ für eine C₁₀- bis C₂₂-Hydrocarbylgruppe steht.

4. Verbindung nach einem der Ansprüche 1 bis 3, wobei der Wert von n im Bereich von 3 bis 7 liegt.

5. Verbindung nach einem der Ansprüche 1 bis 4, bei der es sich um Caprylat/Caprat--(OCH₂CH₂CH₂)₅-isostearyl handelt, wobei in Formel (1) X für R₁C(O) steht, wobei R₁ C₇₋₁₀ ist, n für 5 steht und R für eine Isostearylgruppe steht.

6. Zusammensetzung, umfassend eine Mischung von Verbindungen nach einem der Ansprüche 1 bis 5.

7. Zusammensetzung nach Anspruch 6, wobei die Mischung mindestens Folgendes umfasst:
(i) die Verbindung (en) :
R- (OCH₂CH₂CH₂) ₙ-OR (1a)
und
(ii) die Verbindung (en) :
R₁C(O)-(OCH₂CH₂CH₂)ₙ-OR (1b) .

8. Zusammensetzung nach Anspruch 6 oder 7, wobei das Gewichtsverhältnis, bezogen auf das Gesamtgewicht der Zusammensetzung, zwischen Verbindung(en) der Formel (1a) und Verbindung(en) der Formel (1b) größer als 2:1 ist.

9. Zusammensetzung nach einem der Ansprüche 6 bis 8, ferner umfassend mindestens eine Verbindung der Formel (1c):
R₁C(O)-(OCH₂CH₂CH₂)ₘ-OR₂CO (1c),
wobei:
m für eine ganze Zahl von 2 bis 15 steht,
R₁ und R₂ unabhängig für eine C₂- bis C₃₆-Hydro-carbylgruppe stehen.

10. Zusammensetzung nach Anspruch 9, wobei die Gruppen R₁ und R₂ für C₁₀- bis C₂₂-Hydrocarbylgruppen stehen.

11. Zusammensetzung nach Anspruch 10, wobei R₁ und R₂ für identische Gruppen stehen.

12. Zusammensetzung nach einem der Ansprüche 9 bis 11, wobei der Wert von m im Bereich von 3 bis 7 liegt.

13. Zusammensetzung nach einem der Ansprüche 9 bis 12, wobei der Wert von n und m identisch ist.

14. Zusammensetzung nach einem der Ansprüche 9 bis 13, umfassend weniger als 30 Gew.-% Verbindung(en) der Formel (1c), bezogen auf das Gesamtgewicht der Mischungszusammensetzung.

15. Zusammensetzung nach einem der Ansprüche 6 bis 14, umfassend
(i) mehr als 50 Gew.-% Verbindung (en) der Formel (1a) :
R₁CO- (OCH₂CH₂CH₂) ₙ-OR
und
(ii) weniger als 30 Gew.-% Verbindung(en) der Formel (1b):
R-(OCH₂CH₂CH₂)ₙ-OR,
wobei sich alle Gew.-%-Angaben auf das Gesamtgewicht der Zusammensetzung beziehen.

16. Körperpflegeprodukt, umfassend die Verbindung nach einem der Ansprüche 1 bis 5 oder die Zusammensetzung nach einem der Ansprüche 6 bis 15.

17. Körperpflegeprodukt nach Anspruch 16, wobei das Produkt die Verbindung nach einem der Ansprüche 1 bis 5 oder die Zusammensetzung nach einem der Ansprüche 6 bis 15 als Rheologiemodifikator umfasst.

18. Körperpflegeprodukt nach Anspruch 16 oder Anspruch 17, wobei das Körperpflegeprodukt ferner einen Körperpflege- und/oder Kosmetikwirkstoff umfasst, der einen Wirkstoff umfasst, der aus einem Sonnenschutzmittel, einem Antiperspirant, einem Mittel gegen Alterung, einem feuchtigkeitsspendenden Mittel, einem Mittel zur Pflege des Haars oder der Kopfhaut und einem Färbe- oder Pigmentmittel ausgewählt ist.

19. Verwendung der Verbindung nach einem der Ansprüche 1 bis 5 oder der Zusammensetzung nach einem der Ansprüche 6 bis 15 als sensorisches Mittel und/oder Pigmenbenetzungsmittel in einem Kosmetik- oder Körperpflegeprodukt.

20. Verfahren zur Herstellung einer Verbindung nach einem der Ansprüche 1 bis 5 oder eine Zusammensetzung nach einem der Ansprüche 6 bis 15, wobei das Verfahren Folgendes umfasst:
o einen ersten Schritt des Herstellens einer Mischung von veretherten Polytrimethylenetherglykolen durch Umsetzen von Polytrimethylenetherglykol und einem Alkohol mit der Formel R-OH in Gegenwart eines stark sauren Katalysators und
o einen zweiten Schritt des Veresterns des im ersten Schritt erhaltenen Reaktionsprodukts mit einer Fettcarbonsäure der Formel R₁-COOH in Gegenwart eines stark sauren Katalysators.

## Revendications

1. Composé de la formule suivante 1 :
**X-(OCH₂CH₂CH₂)ₙ-OR** **(1)**
n étant un entier de 2 à 15,
R étant un groupe hydrocarbyle en C₆ à C₃₆,
X étant R ou R₁C(O),
R₁ étant un groupe hydrocarbyle en C₂ à C₃₆, et
les groupes R et R₁ étant des groupes alkyle.

2. Composé selon la revendication 1, le groupe R étant un groupe hydrocarbyle en C₁₀ à C₂₀.

3. Composé ou composition selon la revendication 1 ou 2, le groupe R₁ étant un groupe hydrocarbyle en C₁₀ à C₂₂.

4. Composé selon l'une quelconque des revendications 1 à 3, la valeur de n étant dans la plage de 3 à 7.

5. Composé selon l'une quelconque des revendications 1 à 4, qui est le caprylate/caprate--(OCH₂CH₂CH₂)₅-isostéaryle, dans lequel dans la formule (1), X est R₁C(O), R₁ étant C₇₋₁₀, n étant 5, et R étant un groupe isostéaryle.

6. Composition comprenant un mélange de composés selon l'une quelconque des revendications 1 à 5.

7. Composition selon la revendication 6, le mélange comprenant au moins :
(i) le ou les composés :
**R-(OCH₂CH₂CH₂)ₙ-OR,** **(1a);**
et
(ii) le ou les composés :
**R₁C(O)-(OCH₂CH₂CH₂)ₙ-OR** **(1b).**

8. Composition selon la revendication 6 ou 7, le rapport en poids, sur la base du poids total de la composition, entre le ou les composés de formule (1a) et ou les composés de formule (1b) étant supérieur à 2 : 1.

9. Composition selon l'une quelconque des revendications 6 à 8, comprenant en outre au moins un composé de formule (1c) :
**R₁C(O)-(OCH₂CH₂CH₂)ₘ-OR₂CO** **(1c),**
m étant un entier de 2 à 15,
R₁ et R₂ étant indépendamment un groupe hydrocarbyle en C₂ à C₃₆.

10. Composition selon la revendication 9, les groupes R₁ et R₂ étant des groupes alkyle en C₁₀ à C₂₂.

11. Composition selon la revendication 10, les R₁ et R₂ étant des groupes identiques.

12. Composition selon l'une quelconque des revendications 9 à 11, la valeur de m étant dans la plage de 3 à 7.

13. Composition selon l'une quelconque des revendications 9 à 12, les valeurs de n et m étant identiques.

14. Composition selon l'une quelconque des revendications 9 à 13, comprenant moins de 30 % en poids du ou des composés de formule (1c) par rapport au poids total de la composition de mélange.

15. Composition selon l'une quelconque des revendications 6 à 14 comprenant
(i) plus de 50 % en poids d'un ou de composés de formule (1a) :
**R₁CO-(OCH₂CH₂CH₂)ₙ-OR,**
et
(ii) moins de 30 % en poids d'un ou de composés de formule (1b) :
**R-(OCH₂CH₂CH₂)ₙ-OR,**
tous les % en poids étant basés sur le poids total de la composition.

16. Produit de soin personnel comprenant le composé selon l'une quelconque des revendications 1 à 5 ou la composition selon l'une quelconque des revendications 6 à 15.

17. Produit de soin personnel selon la revendication 16, le produit comprenant le composé selon l'une quelconque des revendications 1 à 5 ou la composition selon l'une quelconque des revendications 6 à 15 en tant que modificateur de rhéologie.

18. Produit de soin personnel selon la revendication 16 ou la revendication 17, le produit de soin personnel comprenant en outre un ingrédient actif de soin personnel et/ou cosmétique comprenant un agent actif choisi parmi un agent de type écran solaire, un agent anti-transpirant, un agent anti-vieillissement, un agent hydratant, un agent de soin capillaire ou du cuir chevelu, et un agent colorant ou pigmenté.

19. Utilisation du composé selon l'une quelconque des revendications 1 à 5, ou de la composition selon l'une quelconque des revendications 6 à 15, en tant qu'agent sensoriel et/ou agent mouillant de pigment dans un produit cosmétique ou de soin personnel

20. Procédé de production d'un composé selon l'une quelconque des revendications 1 à 5 ou d'une composition selon l'une quelconque des revendications 6 à 15, le procédé comprenant :
∘ une première étape de préparation d'un mélange de polytriméthylène éther glycol éthérifié par mise en réaction d'un polytriméthylène éther glycol et d'un alcool possédant la formule R-OH en la présence d'un catalyseur de type acide fort ; et
∘ une deuxième étape d'estérification du produit de réaction obtenu dans la première étape avec un acide carboxylique gras de formule R₁-COOH, en la présence d'un catalyseur de type acide fort.
